(19) **Europäisches Patentamt**

**European Patent Office**

**Office européen des brevets**

(11) **EP 1 067 905 B1**

(12) **EUROPÄISCHE PATENTSCHRIFT**

(45) Veröffentlichungstag und Bekanntmachung des Hinweises auf die Patenterteilung:
**25.09.2002 Patentblatt 2002/39**

(21) Anmeldenummer: **99923373.7**

(22) Anmeldetag: **20.03.1999**

(51) Int Cl.[7]: **A61K 9/00**

(86) Internationale Anmeldenummer:
**PCT/DE99/00798**

(87) Internationale Veröffentlichungsnummer:
**WO 99/049842 (07.10.1999 Gazette 1999/40)**

(54) **BRAUSEFORMULIERUNGEN**

FIZZY FORMULATIONS

FORMULATIONS DE SPRAY

(84) Benannte Vertragsstaaten:
**AT BE CH CY DE DK ES FI FR GB GR IE IT LI LU MC NL PT SE**
Benannte Erstreckungsstaaten:
**LT LV MK RO SI**

(30) Priorität: **31.03.1998 DE 19814257**

(43) Veröffentlichungstag der Anmeldung:
**17.01.2001 Patentblatt 2001/03**

(73) Patentinhaber: **VIATRIS GmbH & Co. KG**
**60314 Frankfurt am Main (DE)**

(72) Erfinder:
• **TRITTHART, Wolfram**
**A-9400 Wolfsberg (AT)**
• **PISKERNIG, Mario, André**
**A-9431 St. Stefan (AT)**
• **KÖLBL, Gottfried**
**A-9100 Völkermarkt (AT)**

(74) Vertreter:
**Wibbelmann, Jobst, Dr., Dipl.-Chem. et al**
**Wuesthoff & Wuesthoff,**
**Patent- und Rechtsanwälte,**
**Schweigerstrasse 2**
**81541 München (DE)**

(56) Entgegenhaltungen:
**EP-A- 0 396 335       WO-A-95/34283**
**DE-A- 3 440 288       US-A- 4 678 661**

EP 1 067 905 B1

**Beschreibung**

Die Erfindung betrifft feste, schnell zerfallende Darreichungsformen in Form von Brauseformulierungen für den alkali-empfindlichen Wirkstoff Selegilin und Verfahren zu ihrer Herstellung.

Selegilin (= L-N-(1-Phenyl-isopropyl)N-methyl-2-propinylamin) oder dessen pharmazeutisch verwendbare Salze werden in der Therapie der Parkinson-Erkrankung in Form von Tabletten eingesetzt.

In der Patentliteratur werden eine Vielzahl von Darreichungsformen für Selegilin beschrieben. In EP 404 807, EP 406 488, EP 509 761, EP 591 432, EP 593 807, EP 617 515, WO 94/23707, EP 647 137, EP 683 668, WO 95/18603, EP 655 900 und WO 96/02239 werden transdermale Applikationsformen, beispielsweise auch in Form von Pflastern beansprucht.

In EP 582 186, WO 96/01612 und US 5,484,608 werden Arzneiformen zur kontrollierten Freisetzung von Selegilin, beispielsweise in Form von Tabletten, in US 5,128,145 osmotisch wirksame Freisetzungssysteme und in WO 96/22435 normal freisetzende orale Formulierungen aufgezeigt.

Gegenstand der Patentanmeldung WO 95/12472 ist eine liposomale Zusammensetzung, welche als Wirkstoff Selegilin enthält.

Buccale und sublinguale Applikationsformen werden in WO 97/17067 beansprucht.

Aus der WO 95/34283 sind Brausegranulate für alkali-empfindliche Arzneistoffe bekannt. Beispielhaft werden in dieser Offenbarung Brausegranulate mit einer Brausebasis aus Zitronensäure, Natriumbicarbonat und Natriumcarbonat erwähnt. In WO 95/07070 werden Brauseformulierungen beschrieben, die zur Vermeidung der unlöslichen Rückstände aus Tricalciumcitrat, die bei der Auflösung von Brauseformulierungen entstehen können, mindestens zwei verschiedene essbare Säuren enthalten.

In WO 93/00886 werden Brausetabletten mit guter Lagerstabilität beispielsweise für die alkali-empfindlichen Wirkstoffe wie Acetylcystein, Captopril und Minoxidil beschrieben, die eine Brausegrundlage bestehend aus einer festen essbaren organischen Säure als Trägerkristalle, einem Alkalicarbonat öder - bicarbonat und einem Alkalisalz der Säure enthalten. Auf den Trägerkristallen sind zwei Schichten aufgebracht. Die erste Schicht besteht aus einer anderen Säure als die Trägerkristalle selbst und die zweite Schicht aus dem Alkalisalz einer der beiden Säuren.

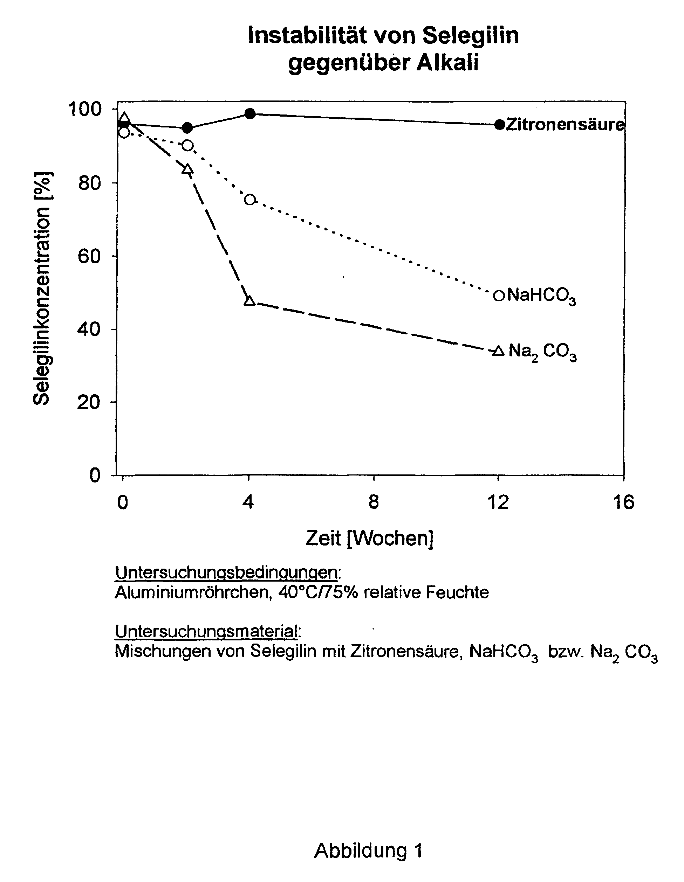

Selegilin ist in alkalihaltigen Brauseformulierungen nicht stabil. Auch ein mehrschichtige Aufbau reicht zur Stabilisierung nicht aus. Alkalicarbonate und bicarbonate sind basischer als Erdalkalicarbonate, wie Calciumcarbonate. Für Selegilin, ein alkali-empfindlicher Wirkstoff, stehen bisher keine geeigneten Brauseformulierungen zur Verfügung. Das dürfte wohl auch daran liegen, daß bisher derartige Formulierungen aufgrund der Instabilität des Selegilins, wie in Abbildung 1 dargestellt, nicht geglückt sind.

Die therapeutische Behandlung von verschiedenen Erkrankungen bedingt, insbesondere bei älteren Menschen, eine sehr häufige und auch teilweise lebenslange Einnahme von Pharmaka.

Ein Parkinson-Patient hat üblicherweise aufgrund des starken Tremors Probleme bei der Einnahme von Tabletten mit dem anschließenden Trinken von Flüssigkeit. Ebenso ist die Einnahme von Tabletten für Patienten mit Schluckbeschwerden sehr schwierig.

Es besteht deshalb ein Bedürfnis nach neuen festen, schnell zerfallenden Darreichungsformen, insbesondere Brauseformulierungen in Form von Löstabletten, Buccaltabletten oder löslichen Granulaten, welche eine einfache Einnahme, beispielsweise auch für ältere Menschen gewährleisten.

Der vorliegenden Erfindung liegt die Aufgabe zugrunde, neuartige und therapeutisch vorteilhafte Brauseformulierungen für Selegilin bereitzustellen.

Insbesondere sind die Löstablettenformulierungen auch für die kombinierte Anwendung mit anderen Löstabletten, wie L-Dopa und Benserazid gemäß EP 521 388 geeignet.

Diese Aufgabe wird durch die vorliegende Erfindung gelöst, welche schnell zerfallende Darreichungsformen zur oralen Anwendung mit oder ohne Wasser in Form von Brauseformulierungen, enthaltend als alkali-empfindlichen Wirkstoff Selegilin oder dessen pharmazeutisch wirksames Salz

und eine Brausegrundlage bestehend aus ein oder mehreren Erdalkalicarbonaten, einer organisch essbaren Säure und/oder einem Alkalisalz der Zitronensäure und gegebenenfalls pharmazeutisch verwendbaren Hilfsstoffen zum Gegenstand hat.

Gegenstand der Erfindung sind Brauseformulierungen in Form von Granulaten, Tabletten oder Sachets. Bei den Tabletten kann es sich auch um Buccaltabletten handeln.

Eine besondere Ausführungsform der Erfindung betrifft Brauseformulierungen, welche Selegilin oder dessen pharmazeutisch verwendbaren Salze enthalten.

Durch Zugabe von Wasser oder Zutritt von Speichel zur derartigen Brausezubereitung entsteht unter Entwicklung von $CO_2$-Gas eine Suspension oder Lösung, welche zur Einnahme einen angenehmen Geschmack besitzt. Eine schnelle Freigabe des Wirkstoffes ist hierbei von besonderer Bedeutung, um einen raschen Wirkungseintritt zu gewährleisten. Dies gilt insbesondere bei Buccaltabletten.

Im Stand der Technik sind Brauseformulierungen für verschiedene Wirkstoffe und Vitamine bekannt. Diese Brauseformulierungen enthalten in der Regel ein $CO_2$ abgabefähiges Mittel sowie ein die Abgabe von $CO_2$-induzierendes Mittel.

Als $CO_2$-abgabefähiges Mittel werden bevorzugt Alkalicarbonate oder Alkalihydrogencarbonate wie Natrium- oder Natriumhydrogencarbonat eingesetzt. Erdalkalicarbonatformulierungen betreffen hauptsächlich Mineralstoffpräparate.

Als Mittel zur Induzierung der $CO_2$-Abgabe werden essbare organische Säuren oder deren Salze, welche in fester Form vorliegen und sich ohne vorzeitige $CO_2$-Entwicklung mit dem Wirkstoff und den anderen Hilfsstoffen zu Granulaten oder Tabletten formulieren lassen, eingesetzt.

Essbare organische Säuren sind beispielsweise Weinsäure, Apfelsäure, Fumarsäure, Adipinsäure, Bernsteinsäure, Ascorbinsäure, Maleinsäure oder Zitronensäure.

Pharmazeutisch annehmbare saure Salze sind beispielsweise in fester Form vorliegende Salze von mehrbasischen Säuren, in denen mindestens noch eine Säurefunktion vorhanden ist, wie Natriumdihydrogen- oder Dinatriumhydrogenphosphat oder entsprechende Citrate.

Die Wirkstoffe liegen entweder als leicht lösliche Verbindungen in der Brauseformulierung vor oder sie werden beim Auflösungsprozeß durch Salzbildung in Lösung gebracht. Schwerlösliche Wirkstoffe können jedoch auch dispergiert werden.

Selegilinhydrochlorid ist außerordentlich empfindlich gegen die üblichen Brausegrundlagen wie Natriumbicarbonat, Natriumcarbonat oder Natriumhydrogencitrat in Verbindung mit organisch essbaren Säuren, wie Zitronensäure oder Weinsäure.

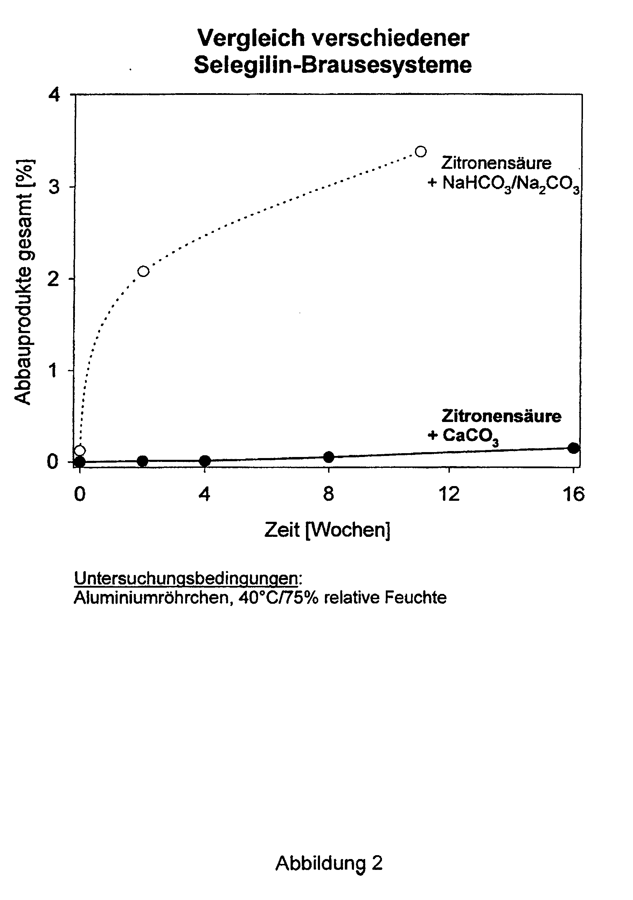

In diesen üblichen Brauseformulierungen kommt es zum Abbau des Wirkstoffes Selegilin zu Amphetamin, Metamphetamin und Desmethylselegilin und zur Sublimation des Wirkstoffes. Bemerkenswert ist, daß der Abbau zu den genannten Metaboliten nur teilweise erfolgt. Der Hauptteil Selegilin sublimiert im Beisein von Alkaliverbindungen, insbesondere Alkalicarbonaten ab, so daß schon überraschenderweise ein Wirkstoffverlust bei geringfügiger Metabolisierung auftritt. Somit ist die geforderte Reinheit und Menge nach Lagerung dieser Selegilin-Brauseformulierungen nicht mehr gegeben, wie aus der Abbildung 2 erkennbar ist.

Überraschenderweise sind die Brauseformulierungen auf Erdalkaligrundlage entsprechend der vorliegenden Erfindung sehr stabil.

Eine bevorzugte Ausführungsform besteht dabei in der Verwendung von Calciumcarbonat und Zitronensäure als Brausegrundlage.

Von Vorteil könnte sein, wenn das Calciumcarbonat teilweise durch die Zitronensäure zu Calciumcitrat anreagiert.

Geringfügige Anteile von Natriumcitrat führen nicht zu Instabilitäten. Dabei darf dieser Anteil 15 % des Gesamtgewichtes der Brauseformulierung nicht übersteigen.

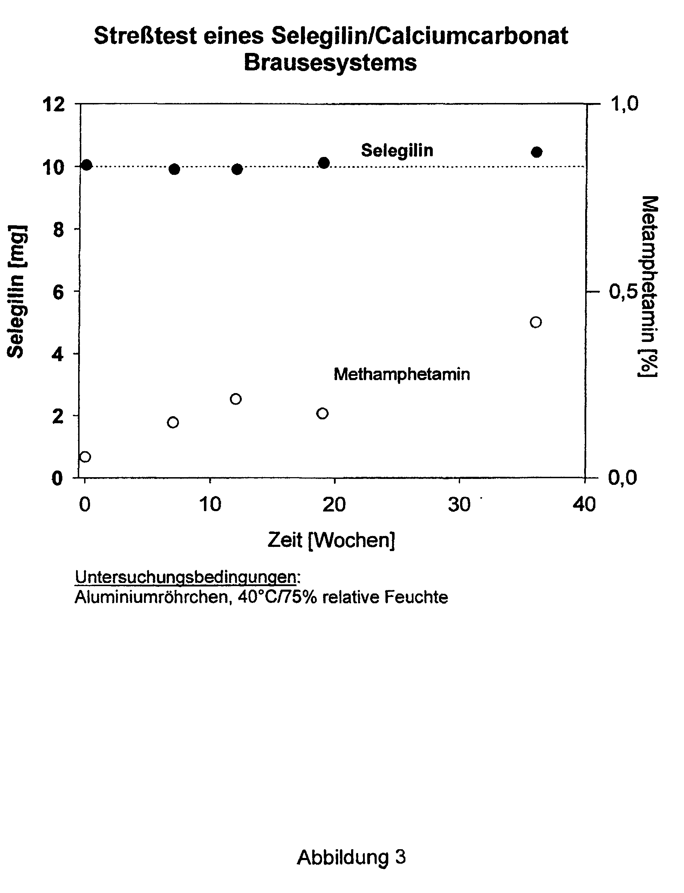

Die erfindungsgemäßen Selegilin-Brauseformulierungen zeigen selbst im Streßtest bei 40 °C und 75 % relativer Luftfeuchtigkeit wie auch bei Raumtemperatur keinen relevanten Qualitätsverlust (Abbildung 3).

Das ist deshalb von besonderer Bedeutung, da Brauseformulierungen während der Herstellung, Abfüllung und Lagerung vor Luftfeuchtigkeit gut geschützt werden müssen, weswegen die Herstellung im allgemeinen nur in Räumen mit niedriger Luftfeuchtigkeit erfolgt (Ritschel, Bio Tablette, Echtio Cauher KG 1966, S. 115 f). Wie von Wells "Pharmaceutical Preformulation (John Wiley 1988) dargelegt, ist die basische Katalyse für sehr viele Arzneistoffe ein entscheidender Mechanismus für Instabilitäten.

Calciumcarbonat wird üblicherweise in Brausetabletten nur zur Calcium-Therapie eingesetzt und nicht als Arzneistoffträger für Wirkstoffgruppen, in denen Calcium keinen Beitrag zur Therapie leistet.

Calciumhaltige Brausetabletten werden im allgemeinen zur Behandlung des Mineralstoffwechsels verwendet.

In WO 95/07070 wird ein Brausegranulat zur Herstellung einer pharmazeutischen Zubereitung auf der Basis von Calciumcarbonat und Zitronensäure aufgezeigt, wobei 5 - 20 Gewichtsteile der Zitronensäure durch wenigstens eine andere essbare Säure, wie Apfelsäure, ersetzt sind.

Calciumcarbonat findet auch als zusätzlicher Hilfsstoff in der pharmazeutischen Technologie, beispielsweise als Dragierhilfsstoff oder Streckmittel Anwendung (Fiedler, Lexikon der Hilfsstoffe, 3. Auflage, 1989).

Die vorliegenden erfindungsgemäßen Brauseformulierungen erlauben die Herstellung einer trinkfertigen, wohlschmeckenden Lösung oder Suspension vorzugsweise in einem Volumen von 40 bis 80 ml Wasser, die bei Tremor leicht abgetrunken werden kann. Dies gilt ebenso für geriatrische Patienten.

Buccal- oder Sublingualbrausezubereitungen werden direkt an der Mundschleimhaut appliziert.

Beispielsweise enthalten Minibrausetabletten 5 bis 10 mg Selegilin-HCl und etwa 1200 mg einer Brausegrundlage, "normale" Brausetabletten 2000 mg bis 7000 mg und Buccalzubereitungen 50 bis 500 mg einer Brausegrundlage. Die Buccalzubereitungen können deutlich niedriger dosiert sein, beispielsweise 1 - 5 mg Selegilin.

Bei niedrig dosierten Wirkstoffen können in den erfindungsgemäßen Brauseformulierungen bis zu 90 % und bei höher dosierten Wirkstoffen 30 % bis 70 % einer Brausegrundlage enthalten sein.

Die Brauseformulierungen erlauben auch die kombinierte Einnahme mit weiteren Wirkstoffen, wie es im Fall von Selegilin bei der Parkinson-Behandlung sehr oft erforderlich ist.

So könnten die Selegilin-Brauseformulierungen in Kombination mit anderen Löstabletten, insbesondere L-Dopa-Benzerazid Kombinationen oder Amantadin-Löstabletten verabreicht werden.

Es ist auch eine Cocktailbehandlung, wie in EP 521 388 beschrieben, möglich. Hier werden wenigstens zwei verschiedene Wirkstoffe in ein und derselben Wassermenge aufgelöst oder suspendiert und damit gemeinsam verabreicht.

Selegilin kann in Form von Brauseformulierungen auch gemeinsam mit Vitamin.E verabreicht werden. Erfindungsgemäß können diese Brausezubereitungen auch für andere alkali-empfindliche Wirkstoffe, wie Erythromycin, Clarithromycin, Diazepam. Ampicillin, oder Phenobarbital eingesetzt werden.

Die Herstellung der erfindungsgemäßen Brauseformulierungen kann nach üblichen, im Stand der Technik bekannten Verfahren erfolgen. Beispielsweise werden die Säuren und Carbonate getrennt granuliert (Feuchtgranulation), wobei die Wirkstoffe vorzugsweise dem sauren Granulat beigefügt werden. Nach Mischung der sorgfältig getrockneten Granulate werden lösliche Gleitmittel, wie Natriumbenzoat oder Polyäthylenglykole zugesetzt und komprimiert.

Nach der anderen Methode werden alle Säuren, Carbonate und Wirkstoffe zusammengemischt und im Reaktor erhitzt, bis beispielsweise die Zitronensäure ihr Kristallwasser freigibt und ein Granulat entsteht (WO95/13130). Wiederholtes Umrühren ist erforderlich, um eine gleichmäßige Masse zu erhalten. Diese wird dann rasch gesiebt und sorgfältig getrocknet. Ein gutes Trocknen ist unbedingt notwendig, um ein allmähliches Zerfallen der Tabletten durch Reaktion der Säuren mit den Carbonaten zu vermeiden.

Um eine rasche Trocknung zu erreichen, werden beispielsweise Vakuumtrockenschränke verwendet.

In einer anderen Variante der Herstellung erfolgt die Anreaktion der Säure mit basischen Bestandteilen mit anschließender Trocknung im Vakuum. Dem trockenen Granulat wird ein lösliches Gleitmittel vor der Kompression beigemischt. Es kann aber auch mit externer Schmierung tablettiert werden.

Das erhaltene erfindungsgemäße Brausegranulat wird weiter zu Tabletten verpreßt oder in Sachets gefüllt.

Vorzugsweise wird der alkali-empfindliche Wirkstoff Selegilin zur Erreichung einer guten Homogenität an neutrale Trägersubstanzen gebunden. Als neutrale Trägersubstanzen für die erfindungsgemäßen Brauseformulierungen werden Lactose, Saccharose, Sorbit, Mannit, Stärke, Pektine oder Cellulosen eingesetzt. Weitere Hilfsstoffe können das Aussehen und/oder die geschmacklichen Eigenschaften der durch Zerfall der Brausetablette erhältlichen wäßrigen Lösungen oder Suspension verbessern, wie Farbstoffe, Zucker oder Süßstoffe.

Die Verwendung von Farbstoffen kann sowohl der Hebung des Aussehens als auch der Kennzeichnung des Präparates dienen. Geeignete für die Verwendung in der Pharmazie zugelassene Farbstoffe sind beispielsweise Carotinoide oder Chlorophylle.

Als Zucker und Süßstoffe können Saccharose, Xylitol, D-Glucose, Sorbitol, Mannitol, Lactose, Aspartame sowie Saccharin-Na, Acesulfam oder Natriumcyclamat verwendet werden.

Die folgenden Beispiele sollen die Erfindung näher erläutern.

| Beispiel 1 | mg | **LÖSTABLETTE** |
|---|---|---|
| Selegilin HCL | 10 | |
| $MgCO_3$ | 96 | |
| $CaCO_3$ | 248 | |
| Zitronensäure | 522 | |
| Aspartame | 4 | |
| Milchzucker | 100 | |
| Aroma | 15 | |
| | 995 | |

| Beispiel 2 | mg | **LÖSTABLETTE** |
|---|---|---|
| Selegilin HCL | 10 | |
| Calciumcarbonat | 310 | |
| Zitronensäure | 620 | |
| Aspartame | 7 | |
| Aroma | 10 | |
| Natriumcitrat | 53 | |
| | 1.010 | |

| Beispiel 3 | mg | LÖSTABLETTE |
|---|---|---|
| Selegilin HCl | 10 | |
| CaCO$_3$ | 380 | |
| Zitronensäure | 500 | |
| Natriumcyclamat | 7 | |
| Saccharin-Natrium | 1 | |
| Aroma | 15 | |
| Yellow 6 | 1 | |
| | 914 | |

| Beispiel 4 | mg | LÖSTABLETTE |
|---|---|---|
| Selegilin | 5 | |
| Calciumcarbonat | 331 | |
| Zitronensäure | 625 | |
| Aspartame | 10 | |
| Aroma | 10 | |
| Natriumcitrat | 19 | |
| | 1.000 | |

| Beispiel 5 | mg | BRAUSEGRANULAT |
|---|---|---|
| Selegilin HCl | 5 | |
| CaCO$_3$ | 410 | |
| Zitronensäure | 600 | |
| Natriumcyclamat | 5 | |
| Saccharin Natrium | 1 | |
| Aroma | 20 | |
| Mannitol | 152 | |
| Aerosil | 2 | |
| Kollidon | 3 | |
| Aspartame | 2 | |
| | 1.200 | |

| Beispiel 6 | mg | BRAUSEGRANULAT |
|---|---|---|
| Selegilin HCl | 10 | |
| CaCO$_3$ | 357 | |
| Zitronensäure | 522 | |
| Natriumcyclamat | 5,7 | |
| Saccharin Natrium | 0,9 | |
| Aroma | 15 | |
| Mannitol | 187 | |
| Aerosil | 2 | |
| Kollidon | 2 | |
| Aspartame | 2 | |
| Yellow 6 | 1 | |
| Natriumcitrat | 100 | |
| | 1.204.6 | |

| Beispiel 7 | mg | **LÖSTABLETTE** |
|---|---|---|
| Selegilin | 5 | |
| $MgCO_3$ | 100 | |
| $CaCO_3$ | 320 | |
| Zitronensäure | 450 | |
| Aspartame | 3 | |
| Milchzucker | 50 | |
| Aroma | 15 | |
| | 943 | |

| Beispiel 8 | mg | **BUCCALTABLETTE** |
|---|---|---|
| Selegilin HCL | 5 | |
| Calciumcarbonat | 250 | |
| Zitronensäure | 112 | |
| Aspartame | 4 | |
| Aroma | 10 | |
| Natriumcitrat | 30 | |
| | 411 | |

| Beispiel 9 | mg | **BUCCALTABLETTE** |
|---|---|---|
| Selegilin HCl | 5 | |
| Calciumcarbonat | 205 | |
| Zitronensäure | 200 | |
| Natriumcyclamat | 2 | |
| Saccharin-Natrium | 0,5 | |
| Aroma | 7 | |
| Mannitol | 71 | |
| Aerosil | 1 | |
| Kollidon | 1,3 | |
| Aspartame | 1 | |
| | 493.8 | |

| Beispiel 10 nicht Bestandteil der Erfindung | mg | **BRAUSEGRANULAT** |
|---|---|---|
| Erythromycin | 500 | |
| $CaCO_3$ | 520 | |
| Zitronensäure | 720 | |
| Natriumcyclamat | 7 | |
| Saccharin-Natrium | 1 | |
| Aroma | 15 | |
| Maisstärke | 60 | |
| Yellow 6 | 1 | |
| | 1.824 | |

| Beispiel 11 nicht Bestandteil der Erfindung | mg | **BRAUSEGRANULAT** |
|---|---|---|
| Diazepam | 5 | |

(fortgesetzt)

| Beispiel 11 nicht Bestandteil der Erfindung | mg | **BRAUSEGRANULAT** |
|---|---|---|
| $MgCO_3$ | 100 | |
| $CaCO_3$ | 320 | |
| Zitronensäure | 450 | |
| Aspartame | 3 | |
| Milchzucker | 50 | |
| Aroma | 15 | |
| | $\underline{\underline{943}}$ | |

**Patentansprüche**

1. Feste, schnellzerfallende Darreichungsformen zur oralen Anwendung in Form von Brauseformulierungen, enthaltend Selegilin oder dessen pharmazeutisch wirksame Salze als Wirkstoff und eine Brausegrundlage bestehend aus ein oder mehreren Erdalkalicarbonaten, einer organisch essbaren Säure und/oder einem Alkalisalz der Zitronensäure und gegebenenfalls pharmazeutisch verwendbaren Hilfsstoffen.

2. Brauseformulierungen nach Anspruch 1 in Form von Löstabletten, Granulaten und Sachets, die vor Einnahme in geeigneten Mengen Wasser gelöst werden.

3. Brauseformulierungen nach Anspruch 1 in Form von Buccal- oder Sublingualtabletten, die direkt in die Mundhöhle appliziert werden.

4. Brauseformulierungen nach Anspruch 1, **dadurch gekennzeichnet, daß** als Brausegrundlagen Calciumcarbonat und/oder Magnesiumcarbonat und Zitronensäure eingesetzt werden.

5. Brauseformulierungen nach den Ansprüchen 1 und 4, **dadurch gekennzeichnet, daß** als Brausegrundlage vorzugsweise Calciumcarbonat und Zitronensäure verwendet wird.

6. Brauseformulierung nach den Ansprüchen 1 und 4, **dadurch gekennzeichnet, daß** die Brausegrundlage bis zu 15% Natriumcitrat enthalten kann.

7. Brauseformulierungen nach Anspruch 1, **dadurch gekennzeichnet, daß** Vitamin E enthalten sein kann.

8. Brauseformulierungen nach Anspruch 1, **dadurch gekennzeichnet, daß** bei niedrig dosiertem Selegilin oder dessen pharmazeutisch wirksamen Salzen bis zu 90% einer Brausegrundlage enthalten sein können.

9. Brauseformulierungen nach Anspruch 1, **dadurch gekennzeichnet, daß** bei höher dosiertem, Selegilin oder dessen pharmazeutisch wirksame Salze 30% bis 70% einer Brausegrundlage enthalten sein können.

10. Brauseformulierungen nach Anspruch 1, **dadurch gekennzeichnet, daß** Minibrausetabletten 5 mg bis 10 mg Selegilin-HCl und 1200 mg einer Brausegrundlage enthalten.

11. Brauseformulierungen nach Anspruch 1, **dadurch gekennzeichnet, daß** Buccalzubereitungen 1-10 mg, insbesondere 5-10 mg Selegilin-HCl und 50-500 mg einer Brausegrundlage enthalten.

12. Brauseformulierungen nach Anspruch 1, **dadurch gekennzeichnet, daß** als zusätzliche Hilfsstoffe Farbstoffe, Zucker und Sißstoffe, wie Saccharose, Xylitol, D-Glucose, Sorbitol, Mannitol, Lactose, Aspartame, Saccharin-Na, Acesulfam oder Natriumcyclamat enthalten sein können.

13. Verfahren zur Herstellung von Brauseformulierungen gemäß Anspruch 1, **dadurch gekennzeichnet, daß** Calcium- und/oder Magnesiumcarbonat und die organisch essbare Säure getrennt granuliert werden, wobei Selegilin oder dessen pharmazeutisch wirksame Salze vorzugsweise dem sauren Granulat beigefügt wird und anschließend die getrockneten Granulate gemischt werden oder alle Bestandteile der Brauseformulierung zusammengemischt werden.

**14.** Verfahren zur Herstellung von Brauseformulierungen nach Anspruch 13, **dadurch gekennzeichnet, daß** zur Erreichung einer guten Homogenität der alkali-empfindliche Wirkstoff an eine neutrale Trägersubstanz gebunden wird.

**15.** Verfahren zur Herstellung von Brauseformulierungen nach Anspruch 14, **dadurch gekennzeichnet, daß** als Trägersubstanzen Lactose, Saccharose, Sorbit, Mannit, Stärke, Pektine oder Cellulosen eingesetzt werden.

**16.** Verfahren zur Herstellung von Brauseformulierungen nach Anspruch 13, **dadurch gekennzeichnet, daß** ein Teil des Calciumcarbonates durch Zitronensäure zu Calciumcitrat anreagiert.

**Claims**

**1.** Solid, rapidly disintegrating administration forms for oral administration in the form of effervescent formulations which comprise selegiline or its pharmaceutically acceptable salts as active ingredient and an effervescent base consisting of one or more alkaline earth metal carbonates, an organic edible acid and/or an alkali metal salt of citric acid and, if appropriate, pharmaceutically acceptable auxiliaries.

**2.** Effervescent formulations according to Claim 1 in the form of soluble tablets, granules and sachets which are dissolved in suitable amounts of water before being taken.

**3.** Effervescent formulations according to Claim 1 in the form of buccal or sublingual tablets which are administered directly into the oral cavity.

**4.** Effervescent formulations according to Claim 1, **characterized in that** the effervescent base used is calcium carbonate and/or magnesium carbonate and citric acid.

**5.** Effervescent formulations according to Claims 1 and 4, **characterized in that** the effervescent base used is preferably calcium carbonate and citric acid.

**6.** Effervescent formulations according to Claims 1 and 4, **characterized in that** the effervescent base may comprise up to 15% of sodium citrate.

**7.** Effervescent formulations according to Claim 1, **characterized in that** they may comprise vitamin E.

**8.** Effervescent formulations according to Claim 1, **characterized in that**, in the case of low-dose selegeline or its pharmaceutically acceptable salts, they may comprise up to 90% of an effervescent base.

**9.** Effervescent formulations according to Claim 1, **characterized in that**, in the case of high-dose selegeline or its pharmaceutically acceptable salts, they may comprise from 30% to 70% of an effervescent base.

**10.** Effervescent formulations according to Claim 1, **characterized in that** effervescent minitablets comprise from 5 mg to 10 mg of selegiline HCI and 1200 mg of an effervescent base.

**11.** Effervescent formulations according to Claim 1, **characterized in that** buccal preparations comprise 1-10 mg, in particular from 5 mg to 10 mg, of selegiline HCI and from 50 to 500 mg of an effervescent base.

**12.** Effervescent formulations according to Claim 1, **characterized in that** it may comprise, as additional auxiliaries, colorants, sugars and sweeteners, such as sucrose, xylitol, D-glucose, sorbitol, mannitol, lactose, aspartame, saccharin-Na, acesulfam or sodium cyclamate.

**13.** Process for preparing effervescent formulations according to Claim 1, **characterized in that** calcium carbonate and/or magnesium carbonate and the organic edible acid are granulated separately, where selegiline or its pharmaceutically acceptable salt is preferably added to the acidic granules and the dried granules are subsequently mixed, or all components of the effervescent formulations are mixed together.

**14.** Process for preparing effervescent formulations according to Claim 13, **characterized in that**, to obtain good homogeneity, the alkali-sensitive active ingredient is bound to a neutral carrier substance.

**15.** Process for preparing effervescent formulations according to Claim 14, **characterized in that** the carrier substances used are lactose, sucrose, sorbitol, mannitol, starch, pectins or celluloses.

**16.** Process for preparing effervescent formulations according to Claim 13, **characterized in that** some of the calcium carbonate has been partially reacted with citric acid to give calcium citrate.


**Revendications**

**1.** Formes de préparations solides se décomposant rapidement pour une application orale dans la forme de formulations effervescentes contenant de la sélégiline ou des sels pharmaceutiquement actifs de celle-ci comme matière active et une base effervescente constituée d'un ou plusieurs carbonates alcalino-terreux, d'un acide organique comestible et/ou d'un sel alcalin de l'acide citrique et éventuellement d'auxiliaires pharmaceutiquement utilisables.

**2.** Formulations effervescentes selon la revendication 1 dans la forme de comprimés à dissoudre, de granulés et de sachets, lesquelles sont dissoutes avant la prise dans une quantité appropriée d'eau.

**3.** Formulations effervescentes selon la revendication 1, dans la forme de comprimés buccaux ou sublinguaux, lesquels sont directement appliqués dans la cavité buccale.

**4.** Formulations effervescentes selon la revendication 1, **caractérisées en ce que** l'on utilise comme supports effervescents du carbonate de calcium et/ou du carbonate de magnésium et de l'acide citrique.

**5.** Formulations effervescentes selon les revendications 1 et 4, **caractérisées en ce que** l'on utilise comme support effervescent de préférence du carbonate de calcium et de l'acide citrique.

**6.** Formulations effervescentes selon les revendications 1 et 4, **caractérisée en ce que** la base effervescente peut contenir jusqu'à 15 % de citrate de sodium.

**7.** Formulations effervescentes selon la revendication 1, **caractérisées en ce qu'**elles peuvent contenir de la vitamine E.

**8.** Formulations effervescentes selon la revendication 1, **caractérisées en ce qu'**elles peuvent contenir jusqu'à 90 % d'une base effervescente pour de la sélégiline faiblement dosée ou des sels pharmaceutiquement actifs de celle-ci faiblement dosés.

**9.** Formulations effervescentes selon la revendication 1, **caractérisées en ce qu'**elles peuvent contenir de 30 % à 70 % d'une base effervescente pour de la sélégiline plus fortement dosée ou des sels pharmaceutiquement actifs de celle-ci plus fortement dosés.

**10.** Formulations effervescentes selon la revendication 1, **caractérisées en ce que** des minicomprimés effervescents contiennent de 5 mg à 10 mg de sélégiline-HCl et 1200 mg d'une base effervescente.

**11.** Formulations effervescentes selon la revendication 1, **caractérisées en ce que** des compositions buccales contiennent 1-10 mg, en particulier 5-10 mg de sélégiline-HCl et 50-500 mg d'une base effervescente.

**12.** Formulations effervescentes selon la revendication 1, **caractérisées en ce qu'**elles peuvent contenir comme auxiliaires supplémentaires des colorants, du sucre et des édulcorants, comme du saccharose, du xylitol, du D-glucose, du sorbitol, du mannitol, du lactose, de l'aspartame, de la saccharine-Na, de l'acésulfame ou du cyclamate de sodium.

**13.** Procédé pour la préparation de formulations effervescentes selon la revendication 1, **caractérisé en ce que** l'on granule séparément du carbonate de calcium et/ou de magnésium et les acides organiques comestibles, de la sélégiline ou des sels pharmaceutiquement actifs de celle-ci étant de préférence ajoutés aux granulats acides et les granulats séchés étant ensuite mélangés ou tous les constituants de la formulation effervescente étant mélangés ensemble.

**14.** Procédé pour la préparation de formulations effervescentes selon la revendication 13, **caractérisé en ce que** la matière active sensible aux alcalis est liée à une substance de support neutre pour atteindre une bonne homogénéité.

**15.** Procédé pour la préparation de formulations effervescentes selon la revendication 14, **caractérisé en ce que** l'on utilise comme substances de support du lactose, du saccharose, du sorbitol, du mannitol, de l'amidon, de la pectine ou des celluloses.

**16.** Procédé pour la préparation de formulations effervescentes selon la revendication 13, **caractérisé en ce qu'**une partie du carbonate de calcium réagit par l'acide citrique pour produire du citrate de calcium.

# Instabilität von Selegilin
# gegenüber Alkali

Untersuchungsbedingungen:
Aluminiumröhrchen, 40°C/75% relative Feuchte

Untersuchungsmaterial:
Mischungen von Selegilin mit Zitronensäure, $NaHCO_3$ bzw. $Na_2CO_3$

Abbildung 1

## Vergleich verschiedener
## Selegilin-Brausesysteme

Untersuchungsbedingungen:
Aluminiumröhrchen, 40°C/75% relative Feuchte

Abbildung 2

Abbildung 3